# EUROPEAN PATENT SPECIFICATION

(11) **EP 3 196 350 B1**
(45) Date of publication and mention of the grant of the patent: **16.02.2022**
(21) Application number: 15836195.6
(22) Date of filing: 26.08.2015
(51) Int. Cl.: D06M 15/356, A61B 5/00, C09D 165/00, C08K 5/42, A61N 1/05, D06M 13/256, D06M 13/275, A61N 1/04, A61B 5/25, A61B 5/291, A61B 5/296, D06M 16/00, D06M 101/12

(54) **ELECTRICALLY CONDUCTIVE MATERIAL AND PRODUCTION METHOD THEREFOR, AND ELECTRODE FOR LIVING BODY**
ELEKTRISCH LEITFÄHIGES MATERIAL UND HERSTELLUNGSVERFAHREN DAFÜR SOWIE ELEKTRODE FÜR LEBENDEN KÖRPER
MATÉRIAU ÉLECTRO-CONDUCTEUR ET SON PROCÉDÉ DE PRODUCTION, ET ÉLECTRODE POUR CORPS VIVANT

(30) Priority: 28.08.2014 JP 2014173595
(43) Date of publication of application: 26.07.2017
(73) Proprietor: Tohoku University, Sendai-shi, Miyagi 980-8577 (JP)
(72) Inventor: TORIMITSU, Keiichi, Sendai-shi Miyagi 980-8577 (JP)
(74) Representative: Plougmann Vingtoft a/s
(86) International application number: PCT/JP2015/074057
(87) International publication number: WO 2016/031872

(56) References cited:
- WO-A1-2013/073673
- JP-A- 2000 173 866
- JP-A- 2007 273 751
- XIA ET AL: "Fabrication and properties of conductive conjugated polymers/silk fibroin composite fibers", COMPOSITES SCIENCE AND TECHNOLOGY, ELSEVIER, AMSTERDAM, NL, vol. 68, no. 6, 19 December 2007 (2007-12-19), pages 1471-1479, XP022527265, ISSN: 0266-3538, DOI: 10.1016/J.COMPSCITECH.2007.10.044
- HO MEI-PO ET AL: "Interfacial bonding and degumming effects on silk fibre/polymer biocomposites", COMPOSITES PART B: ENGINEERING, vol. 43, no. 7, 17 May 2012 (2012-05-17), pages 2801-2812, XP028930862, ISSN: 1359-8368, DOI: 10.1016/J.COMPOSITESB.2012.04.042
- ISABELLA S. ROMERO ET AL: "Enhancing the Interface in Silk-Polypyrrole Composites through Chemical Modification of Silk Fibroin", ACS APPL.MATER.INTERFACES, vol. 5, no. 3, 13 February 2013 (2013-02-13), pages 553-564, XP055390708, ISSN: 1944-8244, DOI: 10.1021/am301844c
- OPWIS KLAUS ET AL.: 'Oxidative in situ deposition of conductive PEDOT:PTSA on textile substrates and their application as textile heating element' SYNTHETIC METALS vol. 162, no. 21-22, 2012, pages 1912 - 1918, XP055360629

## Description

### TECHNICAL FIELD

The present invention relates to an electrically conductive material, a production method for the electrically conductive material, and a bioelectrode.

### BACKGROUND ART

In the field of medical engineering, which is a combination of medical science and engineering, production of alternative devices substituting for physical functions is a challenge being tackled extensively. The production of such devices requires, first and foremost, accurate measurement of biopotentials including, for example, a myoelectric potential. The myoelectric potential may be measured within a subject muscle, as well as from outside of the subject muscle. Such a measurement within a muscle may enable more accurate reproduction of a muscle movement.

In view of this, the present inventor developed a conductive fiber usable as a flexible electrode which is capable of following the movement of a muscle and arranged within the muscle to measure a potential. This conductive fiber is produced by coating a fiber such as a silk fiber that is a biomaterial, with a conductive polymer (see, for example, Patent Documents 1, 2, or Non-Patent Document 1).

The conductive fiber described in each of Patent Document 1 and Non-Patent Document 1 includes poly(3,4-ethylenedioxythiophene)-polystyrenesulfonate (PEDOT-PSS) as a conductive polymer, and is produced by a so-called electrolytic polymerization method. Specifically, according to the electrolytic polymerization method, the conductive fiber is produced in the following manner: A base fiber immersed in a conductive solution containing PEDOT-PSS is allowed to travel between, and be energized by, electrodes while being lifted vertically from the conductive solution, thereby electrochemically polymerizing and fixing the PEDOT-PSS that has been applied to the base fiber. The conductive fiber of Patent Document 2 includes PEDOT-PSS as a conductive polymer and is produced in such a manner that a resin composition as a mixture of PEDOT-PSS and a binder resin is applied to a base fiber, and then solidified or polymerized by drying, warming, or heating, for example.

Note that poly(3,4-ethylene-dioxythiophene)-p-toluenesulfonate (PEDOT-pTS) is known as a conductive polymer which has a higher conductivity than PEDOT-PSS (see, for example, Non-Patent Document 2).

### CITATION LIST

### PATENT DOCUMENT

Patent Document 1: International Publication No. WO 2013/073673
Patent Document 2: Japanese Unexamined Patent Publication No. 2014-108134
Patent Document 3: JP 2007 273751 A
Patent Document 4: JP 2000 173866A
Patent Document 5: WO 2013/073673 A1

### NON-PATENT DOCUMENT

Non-Patent Document 1: S. Tsukada, H. Nakashima, K. Torimitsu, "Conductive polymer combined silk fiber bundle for the bioelectrical signal recording," PLoS ONE, 2012, Vol.7, e33689-1-10
Non-Patent Document 2: Seul Gi Kim, Jong-Won Yang, Jun-Taek Lee, Jin-Yeol Kim, "Highly conductive PEDOT: PTS films interfacially polymerized using electro spray deposition and enhanced by plasma doping," Japanese Journal of Applied Physics, 2014, 53, 035501-1-7
Non-Patent Document 3: XIA ET AL: "Fabrication and properties of conductive conjugated polymers/silk fibroin composite fibers", COMPOSITES SCIENCE AND TECHNOLOGY, ELSEVIER, AMSTERDAM, NL, vol. 68, no. 6, 19 December 2007 (2007-12-19), pages 1471-1479, XP022527265, ISSN: 0266-3538, DOI: 10.1016/J.COMPSCITECH.2007.10.044
Non-Patent Document 4: HO MEI-PO ET AL: "Interfacial bonding and degumming effects on silk fibre/polymer biocomposites", COMPOSITES PART B: ENGINEERING, vol. 43, no. 7, 17 May 2012 (2012-05-17), pages 2801-2812, XP028930862, ISSN: 1359-8368, DOI: 10.1016/J.COMPOSITESB.2012.04.042
Non-Patent Document 5: ISABELLA S. ROMERO ET AL: "Enhancing the interface in Silk-Polypyrrole Composites through Chemical Modification of Silk Fibroin", ACS APPL.MATER.INTERFACES, vol. 5, no. 3, 13 February 2013 (2013-02-13), pages 553-564, XP055390708, ISSN: 1944-8244, DOI: 10.1021/am301844c
Non-Patent Document 6: OPWIS KLAUS ET AL.: "Oxidative in situ deposition of conductive PEDOT:PTSA on textile ubstrates and their application as textile heating element", SYNTHETIC METALS, vol. 162, no. 21-22, 2012, pages 1912-1918, XP055360629

### SUMMARY OF THE INVENTION

### TECHNICAL PROBLEM

The conductive fibers of Patent Documents 1, 2 and Non-Patent Document 1 are pleasant to the touch, highly durable and highly water-resistant, and also have flexibility. Therefore, these fibers may be used as, for example, an electrode arranged within a muscle to measure a potential. However, each of these conductive fibers of the known art, which has the conductive polymer, namely PEDOT-PSS, sparsely applied to the surface of the base fiber, and therefore, has a relatively high resistance value and causes an increased noise when used as an electrode.

Patent document 3 relates to a solid electrolytic capacitor having low ESR, a low leakage current, and a high breakdown voltage, by improving a separator and reducing equivalent series resistance even without heat treatment. The document provide a manufacturing method of the solid electrolytic capacitor. In the solid electrolytic capacitor; formed positive electrode foil and negative electrode foil are wound via a separator , and the separator is allowed to hold conductive polymers. The separator is made of blended paper of paper fiber and cotton. This sort of separator has low density even if no heat-treatment process is made, thus preventing an increase in a low-leakage current and a decrease in a breakdown voltage caused by heat treatment.

Patent document 4 relates to an aluminum electrolytic capacitor which is excellent in electrical properties by a method wherein a leakage current is prevented, and the electrolytic capacitor is restrained in impedance in high frequencies. An anode foil and a cathode foil of aluminum are rolled into a rolled element interposing a separator made of paper, glass paper, or a mixture of paper and glass fiber between them, a silicon-containing film is formed on the rolled element, the rolled element coated with the film is thermally treated for the formation of a capacitor element, the capacitor element is dipped into a solution containing thiophene or its derivative for polymerization. The capacitor element is further impregnated with first conductive high polymer molecule, dipped into a solution which contains thiophene or its derivative or aniline or its derivative to enable polymerization to occur, and impregnated with second conductive high polymer molecule for the formation of an aluminum electrolytic capacitor.

Patent document 5 relates to conductive polymer fibers, a method and a device for producing conductive polymer fibers, a biological electrode, a device for measuring biological signals, an implantable electrode, and another device for measuring biological signals. A conductor containing a conductive polymer impregnates and/or adheres to base fibers, and said conductive polymer is PEDOT-PSS.

Non-patent document 3 relates to highly conductive polymers (polypyrrole, polyaniline or poly3,4-ethylene-dioxythiophene)/silk fibroin composite fibers fabricated by in situ polymerization without any modification of silk fibroin surface. Scanning electron microscopy (SEM) and optical microscope confirmed the silk fibroin fiber surface covered entirely by conductive polymers. Polypyrrole/silk fibroin (PPY/SF), polyaniline/silk fibroin (PANI/SF), and poly3,4-ethylene-dioxythiophene/silk fibroin (PEDOT/SF) composite fibers exhibited varied conductivity in the range of 3.8-4.2 × 10⁻¹, 0.9-1.2 × 10⁻² and 4.9-5.2 × 10⁻³ S cm^{-I}, respectively. Improved thermal stability of silk fibroin fiber via coating process was observed from thermogravimetric analysis (TGA) results. Structural analysis indicated that the interactions including hydrogen-bonding and electrostatic attraction existed between silk fibroin macromolecules and conductive polymers. The peptide linkages bearing on silk fibroin macromolecules played an important role in the in situ polymerization by attracting conjugated cation radicals. These composite fibers still possessed former fibrillar morphology and strength properties.

Non-patent document 4 relates to silk fibre used for bio-medical engineering and surgically-operational applications because of its biocompatible and bioresorbable properties. Using silk fibre as reinforcement for bio-polymers could enhance the stiffness of scaffoldings and bone implants. However, raw silk fibre consists of silk fibroin that is bound together by a hydrophilic glued-liked protein layer called "sericin". Degumming is a surface modification process for sericin removal which allows a wide control of the silk fibre's properties, making the silk fibre possible to be properly used for the development and production of novel bio-composites with specific mechanical and biodegradable properties. Some critical issues such as wettability, bonding efficiency and biodegradability at the fibre/matrix interface are of interesting topics in the study of the degumming process.

Non-patent document 5 relates to production of conductive, biocompatible, and mechanically robust materials for use in bioelectrical applications, by a new strategy to selectively incorporate poly(pyrrole) (Ppy) into constructs made from silk fibroin. The document demonstrate that covalent attachment of negatively charged, hydrophilic sulfonic acid groups to the silk protein can selectively promote pyrrolc absorption and polymerization within the modified films to form a conductive, interpenetrating network of Ppy and silk that is incapable of delamination. To further increase the conductivity and long-term stability of the Ppy network, a variety of small molecule sulfonic acid dopants were utilized and the properties of these silk-conducting polymer composites were monitored over time. The composites were evaluated using attenuated total reflectance Fourier transform infrared spectroscopy (ATR-FTIR), scanning electron microscopy (SEM), optical microscopy, energy-dispersive X-ray (EDX) spectroscopy, *cyclic* voltammetry, a 4-point resistivity probe and mechanical testing. In addition, the performance was evaluated following exposure to several biologically relevant enzymes. Using this strategy, mechanically robust polymer electrodes was produced with stable electrochemical performance and sheet resistivities on the order of I X 10² 1⁻2/sq (conductivity -I S/cm).

In view of the foregoing background, it is therefore an object of the present invention to provide an electrically conductive material which includes a conductive polymer uniformly applied to the surface of a base fiber and has a reduced resistance value, a production method for such an electrically conductive material, and a bioelectrode.

### SOLUTION TO THE PROBLEM

To achieve this object, an electrically conductive material according to the present invention includes: a base consisting of a silk fiber; and poly(3,4-ethylenedioxythiophene)-p-toluenesulfonate (PEDOT-pTS) applied to the base. As used herein, the expression "applied to the base" refers to being applied to the surface of the base, the inside of the base, and both on the surface and inside the base.

In summary, the material for forming the base is not limited to any particular material as long as the material contains sericin or fibroin. The material is silk that contains these proteins by nature.

In general, the material allowed to contain, or be coated or soaked with, sericin or fibroin may be selected from a broad variety of materials, examples of which include: synthetic fibers such as a polyamide fiber, a polyester fiber, an acrylic fiber, an aramid fiber, a polyurethane fiber, and a carbon fiber; vegetable fibers such as a cotton fiber, a linen fiber, and a jute fiber; animal fibers such as the silk fiber descried above, wool, and a collagen fiber; and a fiber blend containing two or more of these fibers. The fiber may be colored.

Among these fibers, the silk fiber is beneficially selected. This is because silk contains sericin or fibroin by nature, has a good affinity for PEDOT-pTS and a high adhesiveness, and possesses a good affinity for a living body and a high strength. This "silk fiber or fiber containing silk as a major ingredient" is the "silk fiber" described above. The silk may be obtained from ordinary domestic silkworms, wild silkworms, or may be made of natural silk deriving from spiders, bees or wasps. Alternatively, the silk fiber may also be obtained by the gene recombination technology. Examples of such recombinant silk include "luminous silk" obtained from a silkworm in which a gene coding a fluorescent protein has been incorporated.

The base is beneficially "linear" or "planar." Being linear refers herein to, for example, having the shape of a thread or a cord, or a vascular bundle having the shape of a fabric or a ribbon. Being planar refers herein to, for example, having the shape of a fabric, a film, a membrane, or a sheet. Apart from the "linear" and "planar" bases, a "gel" base may also be used. A typical example of the linear base is a thread, and a typical example of the planar base is a woven fabric (such as a plain woven fabric or a satin woven fabric).

In the electrically conductive material of the present invention,
(a) if the base is made of a linear member, a conductive portion of the base suitably has an electrical resistance value of 50 kΩ/cm or less, and more suitably of 1.6 kΩ/cm or less, in a cross-sectional area of about 2.5 × 10⁻⁴ cm², and
(b) if the base is made of a member with a different shape from that described in (a), such as a planar member, the conductive portion of the base suitably has an electrical resistance value of 50 kΩ/cm or less, and more suitably of 1.6 kΩ/cm or less.

As used herein, the "conductive portion" refers to a portion of the base subjected to an electroconductive treatment, specifically a portion having PEDOT-pTS applied thereto. If PEDOT-pTS is applied to only a portion of the base, that PEDOT-pTS applied portion corresponds to the "conductive portion."

Regarding the situation (a) mentioned above, as will be described later with respect to examples, the electrical resistance value of the linear electrically conductive material of the present invention is obtained by measuring its electrical resistance per linear length of 1 cm using a tester. The cross-sectional area of about 2.5 × 10⁻⁴ cm² is that of a silk thread having a standard diameter. As used herein, "about" means that "2.5" is given by rounding off the figure to one decimal place. Since the cross-sectional area is inversely proportional to a resistance value, it is easy to calculate a resistance value according to the cross-sectional area. It is expected that the cross-sectional area of PEDOT-pTS increases as the cross-sectional area of a linear base increases. This allows easy comparison between the electrically conductive materials of the present invention or between the electrically conductive material of the present invention and another electrically conductive material. Thus, the cross-sectional area is an appropriate parameter in determining the electrical resistance value in the present invention. If the conductive portion of the electrically conductive material has a length of less than 1 cm, then the electrical resistance value of the entire conductive region of the linear electrically conductive material is measured, and then converted into that of a portion having a length of 1 cm and the above cross-sectional area. Then, the comparison may also be made between them.

Regarding the situation (b) mentioned above, if the base has a nonlinear shape, typically, if the base is made of a planar member such as a fabric, the resistance value cannot be determined based only on the cross-sectional area of the base. However, in general, according to comprehensive resistance conversion of conductors based on their cross-sectional area, a planar member has a larger cross-sectional area than a linear member. Therefore, if the base has a nonlinear shape, the electrical resistance value per length of 1 cm of the conductive portion is determined as "the electrical resistance value per centimeter (Ω/cm or kΩ/cm)." Based on this premise, a suitable range and an optimal range of the linear member described above with respect to the situation (a) may be determined as a feature of the electrically conductive material of the present invention.

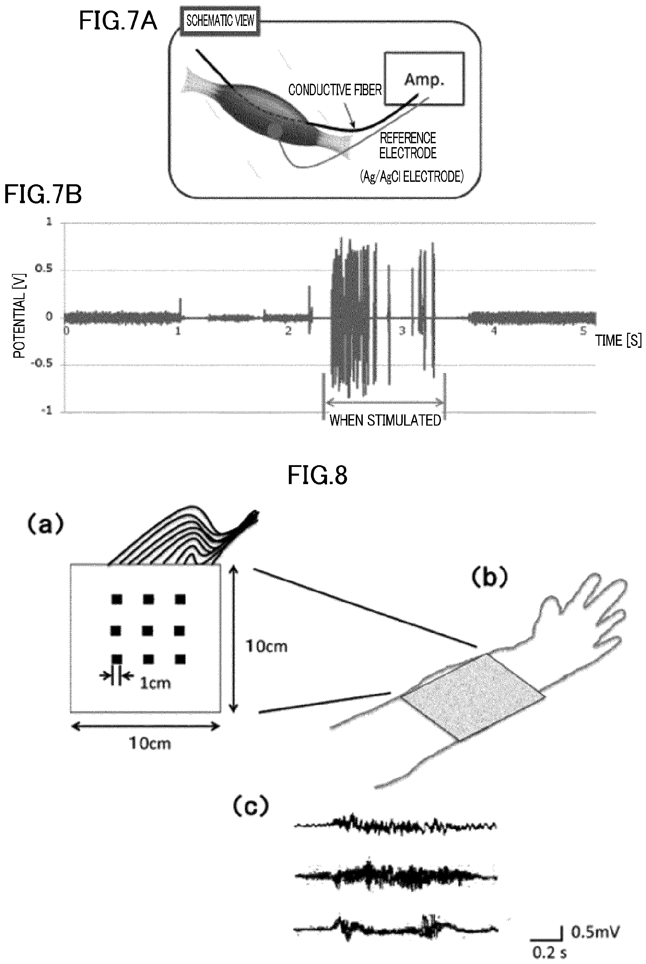

A silk fabric of the example to be described later with reference to FIG. 8 had ten square electrode elements (each 1cm × 1cm), each having an electrical resistance value of less than 1.6 kΩ/cm, which proves the validity of the above-described premise. Like the linear base, if the conductive portion of the electrically conductive material has a length of less than 1 cm, the electrical resistance of the conductive region of the electrically conductive material is measured, and then converted into that of a portion having a length of 1 cm. Then, the comparison may also be made between them.

A method for producing an electrically conductive material of the present invention includes the following steps (1) and (2):
(1) applying a p-toluenesulfonate (pTS) solution containing an oxidant component and pTS to a base selected from the group consisting of a silk fiber, a fiber containing sericin or fibroin, and a fiber coated or soaked with sericin or fibroin; and
(2) further applying 3,4-ethylenedioxythiophene (EDOT) to the base that already has the oxidant component and pTS applied thereto through the step (1), thereby triggering, at the base, a polymerization reaction to form poly(3,4-ethylene-dioxythiophene)-p-toluenesulfonate (PEDOT-pTS) and applying the formed PEDOT-pTS to the base.

The step (1) beneficially includes applying the oxidant component and pTS onto the base by either immersing the base in the pTS solution or printing the pTS solution on the base.

The step (2) suitably includes facilitating the polymerization reaction to form the PEDOT-pTS by heating the pTS solution while applying the EDOT onto the base. It is beneficial to perform, after the step (2), the process step of washing and drying the base to which the PEDOT-pTS has been applied. The base is beneficially washed with water, more beneficially with distilled water or deionized water. The base may be dried in a thermostatic oven, with hot air or warm air, or in the sun. Although drying in the sun is exemplified, it is beneficial to dry the base in a thermostatic oven or with hot or warm air. The thermostatic oven is suitably set at a temperature of 50-80°C, and more suitably at a temperature of 60-70°C for drying the base. If hot or warm air is used, the surface temperature of the target is also suitably at 50-80°C, and more suitably 60-70°C.

Note that pTS and EDOT are both commercially available.

The method for producing the electrically conductive material of the present invention allows PEDOT-pTS, which has a higher degree of conductivity than PEDOT-PSS, to be applied to a base more uniformly and more evenly than the electrolytic polymerization method of the known art, thus achieving a significant reduction in the electrical resistance value of the electrically conductive material. Therefore, when used as an electrode, the electrically conductive material of the present invention may produce a reduced noise. The electrically conductive material produced by the production method according to the present invention has the electrical resistance value described above.

The electrolytic polymerization method of the known art allows almost no PEDOT-pTS to be applied to the surface of a base, and therefore, is unable to turn the surface of the base into an electrically conductive one. In contrast, the method for producing the electrically conductive material of the present invention allows PEDOT-pTS to be applied to the surface of a base uniformly and evenly. As can be seen, the production method of the present invention is suitable for producing the electrically conductive material of the present invention, and may produce the electrically conductive material that has a high degree of electrical conductivity and a lower resistance value.

Note that if PEDOT-PSS were used as the conductive polymer, even the method for producing the electrically conductive material of the present invention could not apply PEDOT-PSS to the base uniformly and evenly. Thus, employing PEDOT-PSS as the conductive polymer would make it difficult to produce an electrically conductive material having a lower resistance value than that produced by the electrolytic polymerization method of the known art.

The base for use in the electrically conductive material of the present invention and for the method for producing the electrically conductive material of the present invention is suitably degummed with an enzyme (mainly a protease).

In the method for producing the electrically conductive material of the present invention, the solution is suitably heated at 50-100°C for 10-60 minutes, more suitably at 50-80°C for 10-40 minutes, and even more suitably at 60-80°C for 10-30 minutes. This heating allows PEDOT-pTS to be applied to the surface of the base faster and more densely, and achieves a further reduction in the resistance value. Further, in the method for producing the electrically conductive material of the present invention, the oxidant component suitably includes transition metal ions. Specifically, the oxidant component suitably includes iron ions, cerium ions, or molybdenum ions, and particularly suitably includes ferric ions. These ions enable efficient formation of PEDOT-pTS.

A bioelectrode according to the present invention includes the electrically conductive material of the present invention.

The bioelectrode includes an element made of the electrically conductive material of the present invention. The element may be designed for a surface electrode or a puncture electrode, for example.

A surface electrode is configured to derive an action potential or a brain wave which has been transmitted via capacity conduction, either through the skin or directly from the surface of a muscle, a brain, or any other organ (such a surface may be hereinafter referred to as "a living body tissue surface"), without having to be needled. The surface electrode is a bioelectrode which is attached to the subject's abdominal muscle or head when it is used. The surface electrode may be configured as an electrode for deriving an evoked potential or as a therapeutic stimulating electrode.

If the electrode of the present invention is configured as such a surface electrode, the electrode includes an electrode element, of which the area contactable with a body tissue surface suitably ranges from 0.0004 cm² to 100 cm², more suitably from 0.0004 cm² to 25 cm². Although an electrode having a contactable area of larger than 25 cm² may also be used as a surface electrode, such an electrode insufficiently takes advantage of a feature of the present invention, that is, "the capability of suitably deriving an action potential, an evoked potential, and a brain wave even with an electrode element having a significantly reduced area contactable with a body tissue surface." A contactable area of smaller than 0.0004 cm² would make it difficult to derive an action potential, an evoked potential, or a brain wave sufficiently. However, if electromyograph systems and electroencephalograph systems have their performance enhanced in the future, an electrode having a contactable area of smaller than 0.0004 cm² could also be used suitably as a surface electrode.

Note that the "area contactable with a body tissue surface" refers herein to the area which is included in the electrically conductive material used as an electrode element for a surface electrode, and which is contactable with a body tissue surface. For example, if the electrode element is planar, the area of the sheet-like electrode element corresponds to the area contactable with the body tissue surface. In the case of such a planar electrode element, in particular, the contactable area suitably ranges from 0.25 cm² to 100cm², and more suitably from 0.25 cm² to 25 cm². If the electrode element is linear, a half of the surface area of the linear electrode element to be in contact with a body surface corresponds to the area contactable with the body tissue surface. The surface area may be approximated, for example, as "the area of a rectangle" one side of which has its length defined by the diameter of the linear material and another side of which has its length defined by the length of the portion to be in contact with the body surface. In the case of such a linear electrode, in particular, the contactable area suitably ranges from 0.0004 cm² to 0.02 cm², and more suitably from 0.0004 cm² to 0.005 cm². Even if the electrode element has a different shape, the electrode element also has an area which may actually be in contact with the body tissue surface and which could be easily determined by those skilled in the art. The contactable area refers herein to the contactable area of a single independent electrode element. For example, if a plurality of electrode elements are arranged separately from each other in a single surface electrode, the contactable area refers to the contactable area of each of those electrode elements.

A puncture electrode literally refers herein to an electrode which is brought into contact with a living body by puncture, and derives a desired biological signal. Examples of the puncture electrode include a needle electrode and a wire electrode. The puncture electrode may be configured as an electrode for deriving an evoked potential or as a therapeutic stimulating electrode. The element of the bioelectrode configured as the puncture electrode suitably has an area contactable with a living body tissue ranging from 0.0004 cm² to 0.02 cm², and more suitably from 0.0004 cm² to 0.002 cm². For a puncture electrode, this contactable area is significantly smaller than contactable areas of the known art. Nevertheless, the electrode of the present invention may still effectively serve as a bioelectrode thanks to its high conductivity.

The electrically conductive material of the present invention, which has a low electrical resistance value, is pleasant to the touch, highly durable and highly water-resistant, and also has flexibility, may be suitably used as a bioelectrode. In particular, the base made of a biomaterial such as a silk fabric and a silk thread allows the electrically conductive material to be suitably used as a bioelectrode. The bioelectrode according to the present invention may be used as an electrode for measuring a potential within a muscle or an electrode for electrocardiogram, for example. As will be described later with respect to examples, the bioelectrode according to the present invention is implementable as a multipoint electrode including two or more electrode elements arranged on a single carrier (e.g. a fabric), which is one of beneficial exemplary embodiments of the present invention.

### ADVANTAGES OF THE INVENTION

The present invention provides an electrically conductive material which includes a base and a conductive polymer applied uniformly and evenly to the surface of the base and has a reduced electrical resistance value. The present invention also provides a producing method for such an electrically conductive material and a bioelectrode.

### BRIEF DESCRIPTION OF THE DRAWINGS

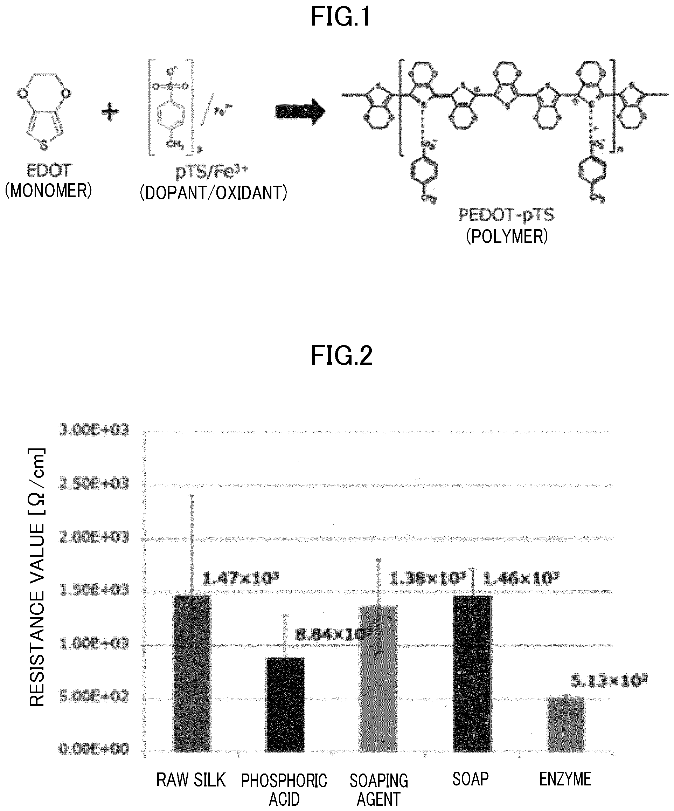

[FIG. 1] FIG. 1 shows a chemical reaction formula representing the formation reaction of PEDOT-pTS in a method for producing an electrically conductive material according to an embodiment of the present invention.
[FIG. 2] FIG. 2 is a graph showing respective electrical resistance values of electrically conductive materials including bases (silk threads) degummed in different ways, and produced by a method for producing an electrically conductive material according to an embodiment of the present invention.

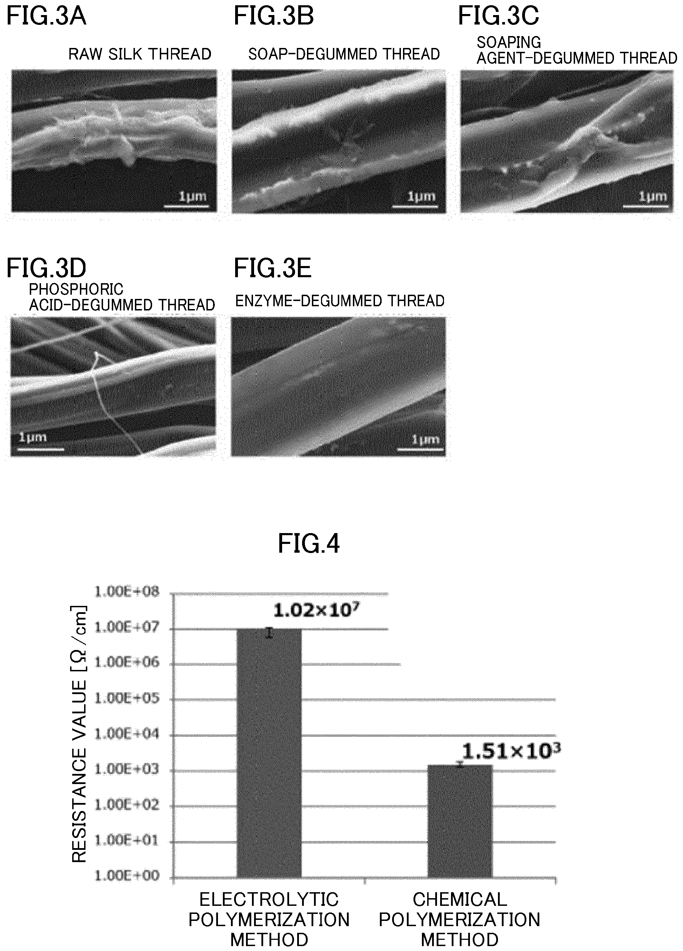

[FIG. 3] FIGS. 3A-3E are electron micrographs of respective electrically conductive materials produced by a method for producing an electrically conductive material according to an embodiment of the present invention. FIG. 3A shows a raw silk thread, and FIG. 3B-3E show silk threads degummed with soap, a soaping agent, phosphoric acid, and an enzyme, respectively.
[FIG. 4] FIG. 4 is a graph showing electrical resistance values of an electrically conductive material produced by a production method (chemical polymerization method) according to an embodiment of the present invention and of an electrically conductive material produced by an electrolytic polymerization method.

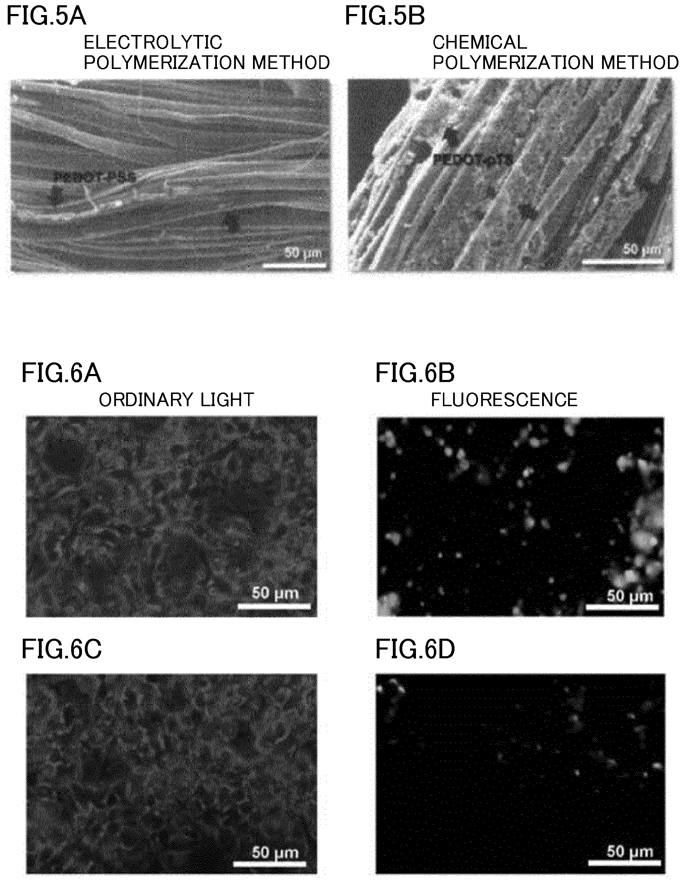

[FIG. 5] FIGS. 5A and 5B are electron micrographs of an electrically conductive material produced by the electrolytic polymerization method, and an electrically conductive material produced by a production method according to an embodiment of the present invention (chemical polymerization method), respectively.
[FIG. 6] FIGS. 6A and 6B are micrographs showing the brain cells of a chick embryo cultured on a cover glass, and observed under ordinary light and fluorescence, respectively. FIGS. 6C and 6D are micrographs showing the brain cells of a chick embryo cultured on a cover glass coated with PEDOT-pTS for use in a method for producing an electrically conductive material according to an embodiment of the present invention, and observed under ordinary light and fluorescence, respectively.
[FIG. 7] FIG. 7A schematically shows how a potential is measured using an electrically conductive material according to an embodiment of the present invention within a muscle of a chick embryo. FIG. 7B is a graph showing a potential measured in the muscle using an electrically conductive material according to an embodiment of the present invention.
[FIG. 8] Portion (a) of FIG. 8 schematically shows a multipoint electrode including the electrically conductive material according to an embodiment of the present invention. Portion (b) of FIG. 8 schematically shows the multipoint electrode attached to a subject. Portion (c) of FIG. 8 is a graph showing potential variations caused by motion and measured on the surface of actual skin.

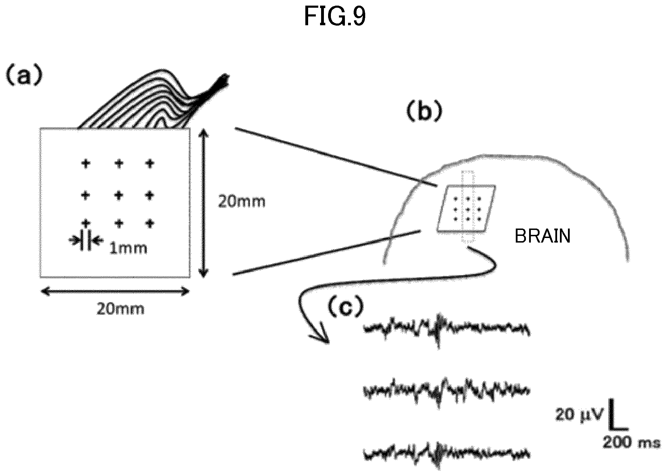

[FIG. 9] Portion (a) of FIG. 9 schematically shows a multipoint electrode including the electrically conductive material according to an embodiment of the present invention and designed for brain wave measurement. Portion (b) of FIG. 9 schematically shows the multipoint electrode attached to the surface of the brain of a chick embryo. Portion (c) of FIG. 9 is a graph showing a brain wave potential measured on the surface of an actual brain.

### DESCRIPTION OF EMBODIMENTS

Embodiments of the present invention will now be described with reference to the drawings.

FIGS. 1-9 show electrically conductive materials according to embodiments of the present invention, and methods for producing the electrically conductive materials.

The electrically conductive material according to each embodiment of the present invention includes a base and PEDOT-pTS applied to the base. The electrically conductive material is produced by one of the production methods according to first and second embodiments of the present invention which will be described below.

Specifically, according to the first embodiment of the present invention, the method for producing the electrically conductive material includes, first, (1) dissolving an oxidant component and pTS as a dopant in an organic solvent-based solution and immersing a silk fabric or a silk thread serving as a base into the organic solvent-based solution.

An organic solvent which allows pTS, an oxidant component, and other components to be dissolved therein is used as the solvent for pTS. The organic solvent for pTS suitably has good compatibility with an aqueous solvent. Specifically, examples of the organic solvents include monovalent lower alcohols with the number of carbon atoms of 1 to 6: namely, methanol, ethanol, propyl alcohol, isopropyl alcohol, butanol, pentanol, and hexanol. The carbon atoms forming each of these monovalent lower alcohols may form a linear skeleton, a branch skeleton, a cyclic skeleton, or a combination of two or more of these skeletons. Each monovalent lower alcohol may be diluted with water, as appropriate, before being used. Among these alcohols, a monovalent lower alcohol with the number of carbon atoms of 1 to 4, namely, methanol, ethanol, propyl alcohol, isopropyl alcohol, or butanol is suitably used as the organic solvent for the pTS solution.

The oxidant component contained in the pTS solution is not limited to any particular substance as long as the oxidant component is capable of activating the polymerization reaction through which pTS and EDOT in contact with each other are polymerized into PEDOT-pTS. Examples of the oxidant components include transition elements and halogens.

Examples of the transition elements include: elements of the first transition series such as iron, titanium, chromium, manganese, cobalt, nickel, and zinc; elements of the second transition series such as molybdenum, silver, zirconium, and cadmium; and elements of the third transition series such as cerium, platinum, and gold. Among these elements, the elements of the first transition series such as iron and zinc are used suitably.

The oxidant component content of the pTS solution varies depending on the type of the oxidant component to be used, and is not limited to any particular content as long as the above-described polymerization reaction can be activated with the oxidant component content. For example, in the case of ferric ions (Fe³⁺) used in an example described herein, they are suitably blended, in the form of ferric chloride, at a ratio of 1-10% by mass, and more suitably at a ratio of 3-7% by mass, with respect to the pTS solution. An excessive amount of ferric chloride blended in the solution would accelerate the polymerization reaction, but would make it difficult to remove iron in a later process step. An insufficient amount of ferric chloride would delay the polymerization reaction.

The content of pTS functioning as a dopant in the pTS solution is suitably 0.1-10% by mass, more suitably 0.15-7% by mass, even more suitably 1-6% by mass, and most suitably 2-5% by mass with respect to the solution.

Next, (2) after EDOT monomers are added to the solution, the solution is heated suitably at 50-100°C for 10-60 minutes, more suitably at 50-80°C for 10-40 minutes, and even more suitably at 60-80°C for 10-30 minutes. After this heating, the base is taken out of the solution, and washed beneficially with water, more beneficially with distilled water or deionized water. Thereafter, the base is dried in a thermostatic oven, with hot air or warm air, in the sun, or in other manners. At room temperature, EDOT is in a liquid state and soluble in water, and may be diluted in an aqueous solvent such as water as appropriate before being used.

The ratio between the amounts of the pTS solution and EDOT (pTS solution:EDOT) in terms of volume ratio ranges from 10:1 to 100:1, and suitably from 20:1 to 40:1.

According to the second embodiment of the present invention, the method for producing the electrically conductive material includes, first, (1) dissolving an oxidant component and pTS as a dopant in an organic solvent-based solution, and print the organic solvent-based solution on a base.

An organic solvent which allows pTS, an oxidant component, and other components to be dissolved therein is used as the solvent for pTS. The organic solvent for pTS suitably has good compatibility with an aqueous solvent. Specifically, examples of the organic solvents include monovalent lower alcohols with the number of carbon atoms of 1 to 6: namely, methanol, ethanol, propyl alcohol, isopropyl alcohol, butanol, pentanol, and hexanol. The carbon atoms forming each of these monovalent lower alcohols may form a linear skeleton, a branch skeleton, a cyclic skeleton, or a combination of two or more of these skeletons. Each monovalent lower alcohol may be diluted with water, as appropriate, before being used. Among these alcohols, a monovalent lower alcohol with the number of carbon atoms of 1 to 4, namely, methanol, ethanol, propyl alcohol, isopropyl alcohol, or butanol is beneficially used as the organic solvent for pTS.

The oxidant component contained in the pTS solution is not limited to any particular substance as long as the oxidant component is capable of activating the polymerization reaction through which pTS and EDOT in contact with each other are polymerized into PEDOT-pTS. Examples of the oxidant component include ions of transition elements and halogens.

Examples of the transition elements include: elements of the first transition series such as iron, titanium, chromium, manganese, cobalt, nickel, and zinc; elements of the second transition series such as molybdenum, silver, zirconium, and cadmium; and elements of the third transition series such as cerium, platinum, and gold. Among these elements, the elements of the first transition series such as iron and zinc are used suitably.

The oxidant component content of the pTS solution varies depending on the type of the oxidant component to be used, and is not limited to any particular content as long as the above-described polymerization reaction is activated with the oxidant component content. For example, in the case of ferric ions (Fe³⁺) used in an example described herein, they are blended, in the form of ferric chloride, suitably at a ratio of 1-10% by mass, and more suitably at a ratio of 3-7% by mass with respect to the pTS solution. An excessive amount of ferric chloride blended in the solution would accelerate the polymerization reaction, but would make it difficult to remove iron in a later process step. An insufficient amount of ferric chloride would delay the polymerization reaction.

The content of pTS functioning as a dopant in the pTS solution is suitably 0.1-10% by mass, and more suitably 0.15-7% by mass, even more suitably 1-6% by mass, and most suitably 2-5% by mass with respect to the solution.

Next, (2) after EDOT monomers are added to the solution, the solution is heated suitably at 50-100°C for 10-60 minutes, more suitably at 50-80°C for 10-40 minutes, and even more suitably at 60-80°C for 10-30 minutes. After this heating, the base is taken out of the solution, and washed beneficially with water, more beneficially with distilled water or deionized water. Thereafter, the base is dried in a thermostatic oven, with hot air or warm air, in the sun, or in other manners. At room temperature, EDOT is in a liquid state and soluble in water, and may be diluted in an aqueous solvent such as water, as appropriate, before being used.

The ratio between the amounts of pTS solution and EDOT (pTS solution:EDOT) in terms of volume ratio ranges from 10:1 to 100:1, and suitably from 20:1 to 40:1.

Either or both of the pTS solution and EDOT that are used in the first and second embodiments of the present invention may contain, as necessary, any other additional components blended therein, provided that such components do not reduce the advantages of the present invention either quantitatively or qualitatively: specifically, provided that such components do not reduce the degree of uniformity of the pTS-EDOT mixture solution applied to the base, or deteriorate the conductivity of the resultant electrically conductive material.

Examples of those additional components include glycerin, polyethylene glycol-polyprene glycol polymer, ethylene glycol, sorbitol, sphingosine, and phosphatidyl choline. Among other things, glycerol, polyethylene glycol-polyprene glycol polymer, and sorbitol are used particularly suitably. Blending these additional components adjusts the wettability property of the electrically conductive material and provides flexibility to the electrically conductive material, which may enhance the affinity for living body tissues, in particular, for skin when the electrically conductive material is used as a bioelectrode. Other examples of the additional components include surfactants, binders, natural polysaccharide, thickeners such as carboxymethylcellulose (CMC), and emulsion stabilizers.

FIG. 1 shows the formation reaction of PEDOT-pTS in the method for producing the electrically conductive material according to the first and second embodiments of the present invention. Note that a silk fabric or a silk thread is used as the base, but the base is not limited to this. Further, as mentioned above, Fe³⁺ is used as an exemplary oxidant component, and the oxidant component of the present invention is not limited to this.

As can be seen from the foregoing, the electrically conductive material of the present invention is suitably produced by the production method of the present invention. The method for producing the electrically conductive material according to embodiments of the present invention includes a heating process step to accelerate the formation reaction of PEDOT-pTS, and consequently, may facilitate the polymerization of PEDOT-pTS with the base. Further, a desired area of the base is immersed in the solution in the process step of immersing the base into the organic solvent-based solution, or the solution is applied, by printing for example, to a desired area of the base in the process step of printing. In this manner, a conductive area may be formed in a desired shape. As a result, the electrically conductive material may be produced which has a conductive area having a shape suitable for its applications and intended operating environment.

The method for producing the electrically conductive material according to each embodiment of the present invention is capable of applying the conductive polymers, i.e., PEDOT-pTS, to the surface of the base more uniformly and evenly than the electrolytic polymerization method or any other method of the known art, thus reducing the electrical resistance value. Further, this method also makes it much simpler, and much less troublesome, to produce the electrically conductive material than the electrolytic polymerization method or any other method of the known art. Such simplicity, which constitutes one of advantageous features of the method (i.e., the chemical polymerization method) for producing the electrically conductive material according to the embodiments of the present invention, allows a fiber to be turned into an electrically conductive one through a process such as printing or spraying which has been impractical according to the electrolytic polymerization method.

The electrically conductive material according to each embodiment of the present invention is pleasant to the touch, highly durable and highly water-resistant, and also has flexibility, a low electrical resistance value, and high biocompatibility. Therefore, the electrically conductive material may be used, for example, for a wearable electrode for healthcare, an electrode for measuring a potential within a muscle or on a skin surface, an electrode for measuring an electrocardiogram, an electrode for measuring an electroencephalography, and a bioelectrode for use in clinical therapy.

The electrically conductive material according to each of the embodiments of the present invention was tested on electrical resistance value, biocompatibility, and other properties. The test results as well as evaluation and studies made on the test results are set forth below.

### [Study on Base]

Several bases including silk threads degummed in different ways were tested to determine whether the difference in the degumming way caused to the resultant electrically conductive materials to have different resistance values. Each of the silk threads under test included eight silk fibers having a size of 21 denier and had a size of 168 denier (with a cross-sectional area of about 2.5 × 10⁻⁴ cm²). The silk threads under test were of the five types of: raw silk thread (hereinafter referred to as non-degummed), a phosphoric acid-degummed thread (acid degummed), a soaping agent-degummed thread (soaping agent degummed), a soap degummed thread (alkaline degummed), and an enzyme-degummed thread (degummed using protease).

Each of the five types of the silk threads was formed into an electrically conductive material by the production method according to the embodiment of the present invention described below. Specifically, 6.3 ml of butanol solution (product of Heraeus K.K.; product name "CLEVIOS^{®} C-B 40 V2"; containing about 4% by mass of p-toluene sulfonic acid iron (III)) was prepared such that the butanol solution contained pTS and ions of ferric (III) that is a transition metal. Each silk thread was immersed in the prepared solution. Next, 220 µl of EDOT (product of Heraeus K.K., product name "CLEVIOS^{®} MV2"; containing about 98.5 % by mass of EDOT) was added to the solution. Thereafter, the solution was heated at 50-100°C for 10-60 minutes in a thermostatic oven. After the heating, each thread was taken out of the solution. Each thread was then washed three times with deionized water, and subsequently, dried in a thermostatic oven at 70°C. Among the electrically conductive materials produced under these conditions, ones prepared by being heated at 70°C for 20 minutes were subjected to the following tests focusing on the differences in degumming way.

Each of the electrically conductive materials formed of the above silk threads was subjected to resistance value measurement. FIG. 2 shows the results of the measurement. As shown in FIG. 2, it was confirmed that the electrically conductive material formed of the enzyme-degummed thread had the lowest resistance value, and the electrically conductive material formed of the phosphoric acid-degummed thread also had a low resistance value. Here, the electrical resistance value refers to the electrical resistance value per centimeter of each of those threads, measured using a tester (the same applies to the following).

FIGS. 3A-3E are electron micrographs of the surfaces of the silk threads degummed in different ways. As shown in FIGS. 3A-3E, the surface of the enzyme-degummed thread is the smoothest, and the surface of the phosphoric acid-degummed thread is also relatively smooth. This suggests that the good surface condition of the enzyme-degummed thread and the phosphoric acid-degummed thread is a factor in the low resistance value of the electrically conductive material. That is to say, forming an electrically conductive material using a base having a smooth surface such as the enzyme-degummed thread or the phosphoric acid-degummed thread would allow PEDOT-pTS to be applied densely to the surface of the base and reduce the resistance value.

### [Production Method]

Note that the following is merely an example, and the present invention is not intended to be limited to this.

The electrically conductive material produced by the method for producing electrically conductive material disclosed in [Study on Base] above (hereinafter referred to as "chemical polymerization method") was compared with an electrically conductive material produced by the electrolytic polymerization method as disclosed in Patent Document 1 and Non-Patent Document 1. Both of these electrically conductive materials included, as the base, the raw silk thread used in [Study on Base].

One of these two types of electrically conductive materials was produced by the electrolytic polymerization method as described in Patent Document 1 and Non-Patent Document 1. Specifically, the raw silk thread was kept immersed for one night in a mixture solution obtained by adding EDOT at a mass ratio of 0.1% to a PEDOT-PSS solution. Next, an Ag/AgCl electrode as a reference electrode and a Pt electrode as a counter electrode were immersed in the mixture solution, and both ends of the raw silk thread were pinched by a clip connected to a working electrode, while a middle portion of the raw silk thread kept immersed in the mixture solution. A potential for polymerization (of 0.8V vs. Ag/AgCl) was applied to both ends of the raw silk thread using a potentiostat to perform the electrolytic polymerization until the total potential reached 76.8 µC. Thereafter, the raw silk thread was lifted out of the mixture solution, and then dried in a thermostatic oven at 70°C.

The other type of electrically conductive material was produced by the chemical polymerization method using the raw silk thread heated at 70°C for 20 minutes, i.e., under one of the heating conditions described for [Study on Base] above.

The resistance values of the two types of electrically conductive materials produced by the chemical polymerization method and the electrolytic polymerization method, respectively, were measured, and the results are shown in FIG. 4. As shown in FIG. 4, it was confirmed that the resistance value of the electrically conductive material produced by the chemical polymerization method is lower by about four orders of magnitude than that of the electrically conductive material produced by the electrolytic polymerization method. As can be seen, the electrically conductive material produced by the chemical polymerization method, having a high degree of conductivity and a lower resistance value, enables noise reduction and more accurate measurement when used as an electrode.

FIGS. 5A and 5B are electron micrographs of the electrically conductive material produced by the electrolytic polymerization method and the electrically conductive material produced by the chemical polymerization method, respectively. As shown in FIG. 5A, it was confirmed that the electrically conductive material produced by the electrolytic polymerization method has PEDOT-PSS applied locally to parts of the surface of the base. In contrast, as shown in FIG. 5B, it was confirmed that the electrically conductive material produced by the chemical polymerization method has PEDOT-pTS applied uniformly and evenly over the entire surface of the base. It is a general understanding that conductivity increases and the resistance value decreases with increase in the surface area of PEDOT covering the surface of a base. Thus, the difference in the degree of uniformity of the applied PEDOT shown in FIGS. 5A and 5B seems to have caused the difference in resistance value.

### [Study on Biocompatibility]

The electrically conductive material according to the embodiment of the present invention was tested on biocompatibility. First, the surface of a cover glass was spin-coated with PEDOT-pTS dripped on the surface, thereby forming a thin film of PEDOT-pTS thereon. Primary culture of brain cells of a chick embryo was conducted on the thin film. In this culture, the brain of the chick embryo was soaked in a trypsin solution for about 10 minutes, and then, the solution was agitated about 10 times using a pipette. Thereafter, 300 µl of the solution was transferred to a 35 mm culture plate, and 2 ml of a culture solution comprised of Neurobasal medium, including 2% B27 supplement, 0.074 mg/ml of L-glutamine, 25 µM of glutamate, and 20 ng/ml of NGF, was added to the culture plate. The culture plate was then placed in an incubator at 37°C and cultured for 24 hours. After the completion of the culture, the cells were stained with 5 µg/ml of Acridine Orange, a fluorescent dye. The cells were then excited with Ar laser to determine viability. A dead cell does not eject the fluorescent dye from itself, and fluorescence is observed. For comparison, cells were cultured using a cover glass not spin-coated with PEDOT-pTS, and observed for fluorescence.

FIGS. 6A-6D show the results. FIGS. 6A and 6B are micrographs showing the brain cells of the chick embryo cultured on the cover glass, and observed under ordinary light and fluorescence, respectively. FIGS. 6C and 6D are micrographs showing the brain cells of the chick embryo cultured on the cover glass coated with the PEDOT-pTS for use in the method for producing the electrically conductive material according to the embodiment of the present invention, and observed under ordinary light and fluorescence, respectively. As shown in FIG. 6B, it was confirmed that on the cover glass not spin-coated with PEDOT-pTS, many cells emitted fluorescence, i.e., many cells were dead. In contrast, as shown in FIG. 6D, it was confirmed that on the cover glass spin-coated with PEDOT-pTS, almost no fluorescence was observed, which means that the cultured cells were alive. These results demonstrate that PEDOT-pTS has high biocompatibility and causes almost no rejection in living body's tissues.

Next, as shown in FIG. 7A, a potential was measured within a muscle of a chick embryo. Specifically, in the potential measurement, the electrically conductive material according to the embodiment of the present invention, which included a raw silk thread as the base and PEDOT-pTS applied to the surface of the base, was used as an element of a bioelectrode. The electrically conductive material was produced using the raw silk thread heated at 70°C for 20 minutes, i.e., under one of the heating conditions described for [Study on Base] above. The measurement results are shown in FIG. 7B. As shown in FIG. 7B, it was confirmed that the electrically conductive material was capable of measuring a potential variation which occurred when the muscle was moved by stimulation.

### [Study (1) on Multipoint Surface Electrode]

Study on this surface myoelectric potential measurement is shown in the schematic diagrams and the potential measurement chart of FIG. 8.

A ribbon-shaped silk fabric having a width of 1 cm and a length of 10 cm (a plain-woven silk fabric made of silk threads degummed with an enzyme (i.e., a protease)) was used as the base, and heated at 70°C for 20 minutes, i.e., under one of the heating conditions of the production method (the chemical polymerization method) disclosed in [Study on Base], thereby producing an electrically conductive material. The electrically conductive material thus produced was cut into square pieces measuring 1 cm on each side (and having an area of 1 cm²). In this manner, ten surface electrode elements having this size were produced. The electrical resistance value between both ends of each of these ten flat plate-shaped pieces of the electrically conductive material was measured with a tester. All of the pieces had a resistance value of less than 1.6 kΩ. A linear electrode element made of a raw silk thread (non-degummed) also heated at 70°C for 20 minutes as described in [Study on Base] was sewn on and connected to each of the surface electrode elements, thereby producing unit elements for a multipoint surface electrode. By using a needle, the linear portion of each unit element was made to perpendicularly penetrate a silk fabric (a plain-woven silk fabric made of silk threads degummed with an enzyme (i.e., a protease)) for use as an insulator, and then sewn on the silk fabric in a simple manner. The silk fabric had a square shape measuring 10 cm on each side (and having an area of 100 cm²). The nine unit elements were sewn on a surface of the silk fabric at regular intervals of 0.5 cm. In this manner, the multipoint surface electrode (see portion (a) of FIG. 8) was produced.

Next, the multipoint surface electrode was fixed on an arm of a subject such that the surface on which the surface electrode elements were exposed was in contact with the subject's arm. Potential variations caused by the motion of the subject's hand were measured with a commercially available wireless electromyography (ID3PAD: product of Oisaka Electronic Equipment Ltd.) (see portion (b) of FIG. 8). In this measurement, the multipoint surface electrode was brought into direct contact with the skin, without any substance for reducing impedance, such as gel, applied between the multipoint surface electrode and the skin.

### [Study (2) on Multipoint Surface Electrode]

Study on this surface brain wave measurement is shown in the schematic diagrams and the potential measurement chart of FIG. 9.

Silk threads having a diameter of 0.2 mm (and degummed with an enzyme (i.e., a protease)) were heated at 70°C for 20 minutes, i.e., under one of the heating conditions of the production method (the chemical polymerization method) disclosed for [Study on Base], thereby producing linear electrode elements each including the silk thread as the base. A silk fabric (a plain-woven silk fabric made of silk threads degummed with an enzyme (i.e., a protease)) having a square shape measuring 20 mm on each side (and having an area of 400 mm²) was provided for use as an insulator. One linear electrode element was made to perpendicularly penetrate the silk fabric from the back of the silk fabric. The tip end of the linear electrode element that had penetrated the silk fabric was then again made to penetrate the silk fabric from the front such that a portion of the linear electrode element with a length of 1 mm was exposed on the front. In this manner, the linear electrode was sewn and fixed as a first liner electrode element. Subsequently, another portion of the linear electrode element was also sewn and fixed in the proximity of the sewn and fixed portion of the first linear electrode element such that another portion intersected at right angles with the 1 mm exposed portion of the first linear electrode element. These two portions of the linear electrode element intersecting with each other at right angles formed a set of unit elements for a multipoint surface electrode. Each set of unit elements, which included two 1 mm portions of the silk thread having a diameter of 0.2 mm, had an electrode area of 0.004 cm². Nine sets of unit elements were sewn at nine points arranged at regular intervals of 5 mm on the square fabric surface measuring 20 mm on each side. In this manner, a multipoint surface electrode for brain wave measurement was produced (see portion (a) of FIG. 9).

Next, the electrode surface of the multipoint surface electrode for brain wave measurement was brought into contact with the surface of the brain of a chick embryo (19 day old) from which the cranial bones had been removed. A commercially available RZ5 bio amp processor (product of Tucker-Davis Technologies, Inc.) was wired to the multipoint surface electrode to measure cranial nerve activity (see portion (b) of FIG. 9). In the measurement, the multipoint surface electrode was brought into direct contact with the brain, without any substance for reducing impedance, such as gel, applied between the multipoint surface electrode and the skin.

As described above, the electrically conductive material according to the embodiment of the present invention has high bio compatibility, and is sufficiently useful as a bioelectrode. In particular, if the base includes silk fibers, the electrically conductive material may be used suitably as a bioelectrode.

## Claims

1. An electrically conductive material comprising:
a base consisting of a silk fiber degummed with an enzyme; and poly(3,4-ethylene-dioxythiophene)-p-toluenesulfonate (PEDOT-pTS) applied to the base.

2. The electrically conductive material of claim 1, wherein
the base has the shape of a thread, a cord, a fabric, a film, a membrane or a sheet, or a vascular bundle having the shape of a fabric or a ribbon.

3. A bioelectrode comprising the electrically conductive material of claim 1 or 2.

4. The bioelectrode of claim 3, wherein
the bioelectrode is configured as a surface electrode, and includes a planar electrode element of which an area contactable with a living body tissue ranges from 0.25 cm² to 100 cm².

5. The bioelectrode of claim 3, wherein
the bioelectrode is configured as a surface electrode, and includes a linear electrode element of which an area contactable with a living body tissue ranges from 0.0004 cm² to 0.002 cm².

6. The bioelectrode of claim 3, wherein
the bioelectrode is configured as a puncture electrode, and includes an electrode element of which an area contactable with a living body tissue ranges from 0.0004 cm² to 0.002 cm².

7. The bioelectrode of any one of claims 3 to 6, wherein
the bioelectrode is configured as a multipoint electrode.

8. A method for producing an electrically conductive material, the method comprising the steps of:
(1) applying a p-toluenesulfonate (pTS) solution containing an oxidant component and pTS to a base consisting of a silk fiber degummed with an enzyme by immersing the base into the pTS solution; and
(2) further applying 3,4-ethylenedioxythiophene (EDOT) to the base that already has the oxidant component and pTS applied thereto through the step (1) and that remains immersed in the pTS solution, while heating the pTS solution at 50-100°C for 10-60 minutes, thereby triggering, at the base, a polymerization reaction to form poly(3,4-ethylenedioxythiophene)-p-toluenesulfonate (PEDOT-pTS) and applying the formed PEDOT-pTS to the base; and wherein the oxidant component includes ferric ions.

9. The method of claim 8, further comprising, after the step (2), the step of washing and drying the base to which the PEDOT-pTS has been applied.

## Patentansprüche

1. Elektrisch leitfähiges Material umfassend:
eine Basis, die aus einer mit einem Enzym entschleimten Seidenfaser besteht; und
Poly(3,4-Ethylen-Dioxythiophen)-p-Toluolsulfonat (PEDOT-pTS), das auf die Basis aufgebracht ist.

2. Elektrisch leitfähiges Material nach Anspruch 1, wobei
die Basis die Form eines Fadens, einer Schnur, eines Gewebes, eines Films, einer Membran oder einer Platte, oder eines Gefäßbündels mit der Form eines Gewebes oder eines Bandes hat.

3. Bioelektrode, die das elektrisch leitfähige Material nach Anspruch 1 oder 2 umfasst.

4. Bioelektrode nach Anspruch 3, wobei
die Bioelektrode als Flächenelektrode konfiguriert ist und ein Flächenelektrodenelement aufweist, dessen mit einem lebenden Körpergewebe in Kontakt bringbare Fläche im Bereich von 0,25 cm² bis 100 cm² liegt.

5. Bioelektrode nach Anspruch 3, wobei
die Bioelektrode als Flächenelektrode konfiguriert ist und ein Linearelektrodenelement aufweist, dessen mit einem lebenden Körpergewebe in Kontakt bringbare Fläche im Bereich von 0,0004 cm² bis 0,002 cm² liegt.

6. Bioelektrode nach Anspruch 3, wobei
die Bioelektrode als Einstichelektrode konfiguriert ist und ein Elektrodenelement aufweist, dessen mit einem lebenden Körpergewebe in Kontakt bringbare Fläche im Bereich von 0,0004 cm² bis 0,002 cm² liegt.

7. Bioelektrode nach einem der Ansprüche 3 bis 6, wobei die Bioelektrode als Mehrpunktelektrode konfiguriert ist.

8. Verfahren zur Herstellung eines elektrisch leitfähigen Materials, wobei das Verfahren die Schritte umfasst von:
(1) Aufbringen einer p-Toluolsulfonat- (pTS) Lösung, die eine oxidierende Komponente und pTS enthält, auf eine Basis, die aus einer mit einem Enzym entschleimten Seidenfaser besteht, durch Eintauchen der Basis in die pTS-Lösung; und
(2) weiter Aufbringen von 3,4-Ethylendioxythiophen (EDOT) auf die Basis, auf die bereits die oxidierte Komponente und pTS durch den Schritt (1) aufgebracht wurden und die in die pTS-Lösung eingetaucht bleibt, während die pTS-Lösung 10-60 Minuten lang auf 50-100 °C erwärmt wird, wodurch an der Basis eine Polymerisationsreaktion ausgelöst wird, um Poly(3,4-EthylenDioxythiophen)-p-Toluolsulfonat (PEDOT-pTS) zu bilden, und Aufbringen des gebildeten PEDOT-pTS auf die Basis; und wobei die oxidierende Komponente Eisen-Ionen aufweist.

9. Verfahren nach Anspruch 8, weiter umfassend, nach dem Schritt (2), den Schritt des Waschens und des Trocknens der Basis, auf die das PEDOT-pTS aufgebracht wurde.

## Revendications

1. Matériau électro-conducteur comprenant :
une base consistant eu une fibre de soie dégommée à l'aide d'une enzyme ; et
du poly(3,4-éthylène-dioxythiophène)-p-toluènesulfonate (PEDOT-pTS) appliqué sur la base.

2. Matériau électro-conducteur selon la revendication 1, dans lequel
la base a la forme d'un fil, d'un cordon, d'un tissu, d'un film, d'une membrane ou d'une feuille, ou d'un faisceau vasculaire ayant la forme d'un tissu ou d'un ruban.

3. Bioélectrode comprenant le matériau électro-conducteur selon la revendication 1 ou 2.

4. Bioélectrode selon la revendication 3, dans laquelle
la bioélectrode est configurée sous forme d'une électrode de surface, et inclut un élément d'électrode plan dont une surface apte à entrer en contact avec un tissu corporel vivant se situe dans la plage de 0,25 cm² à 100 cm².

5. Bioélectrode selon la revendication 3, dans laquelle
la bioélectrode est configurée sous forme d'une électrode de surface et inclut un élément d'électrode linéaire dont une surface apte à entrer en contact avec un tissu corporel vivant se situe dans la plage de 0,0004 cm² à 0,002 cm².

6. Bioélectrode selon la revendication 3, dans laquelle
la bioélectrode est configurée sous forme d'une électrode ponctuelle et inclut un élément d'électrode dont une surface apte à entrer en contact avec un tissu corporel vivant se situe dans la plage de 0,0004 cm² à 0,002 cm².

7. Bioélectrode selon l'une quelconque des revendications 3 à 6, dans laquelle la bioélectrode est configurée sous forme d'une électrode multipoint.

8. Procédé de production d'un matériau électro-conducteur, le procédé comprenant les étapes consistant à :
(1) appliquer une solution de p-toluènesulfonate (pTS) contenant un composant oxydant et du pTS sur une base consistant en une fibre de soie dégommée à l'aide d'une enzyme, par immersion de la base dans la solution de pTS ; et
(2) appliquer en outre du 3,4-éthylènedioxythiophène (EDOT) sur la base sur laquelle sont déjà appliqués le composant oxydant et du pTS par l'étape (1) et qui reste immergée dans la solution de pTS, tout en chauffant la solution de pTS à 50-100 °C pendant 10-60 minutes, ce qui déclenche, au niveau de la base, une réaction de polymérisation conduisant à former du poly(3,4-éthylènedioxythiophène)-p-toluènesulfonate (PEDOT-pTS) et applique sur la base le PEDOT-pTS formé ; et dans lequel le composant oxydant inclut des ions ferriques.

9. Procédé selon la revendication 8, comprenant en outre, après l'étape (2), l'étape consistant à laver et sécher la base sur laquelle le PEDOT-pTS a été appliqué.
